# EUROPÄISCHE PATENTSCHRIFT

(11) **EP 3 077 013 B1**
(45) Veröffentlichungstag und Bekanntmachung des Hinweises auf die Patenterteilung: **10.01.2018**
(21) Anmeldenummer: 14786690.9
(22) Anmeldetag: 22.10.2014
(51) Int. Cl.: A61L 2/28, A61B 50/00, F16J 15/16

(54) **STERILANZEIGE FÜR EINEN STERILBEHÄLTER**
STERILIZATION INDICATOR FOR A STERILE CONTAINER
INDICATEUR DE STÉRILITÉ POUR RÉCIPIENT STÉRILE

(30) Priorität: 30.10.2013 DE 102013111979
(43) Veröffentlichungstag der Anmeldung: 12.10.2016
(73) Patentinhaber: Aesculap AG, 78532 Tuttlingen (DE)
(72) Erfinder: SCHULZ, Peter, 79843 Löffingen (DE); SCHUSTER, Stefan, 79865 Grafenhausen (DE); THOMAS, Stefan, 78532 Tuttlingen (DE)
(74) Vertreter: Winter, Brandl, Fürniss, Hübner, Röss, Kaiser, Polte - Partnerschaft mbB
(86) Internationale Anmeldenummer: PCT/EP2014/072616
(87) Internationale Veröffentlichungsnummer: WO 2015/062924

(56) Entgegenhaltungen:
- EP-A1- 0 849 429
- EP-B1- 0 412 571
- WO-A2-2013/060667
- DE-U1-202009 010 210
- US-A1- 2005 183 656

## Beschreibung

Die Erfindung betrifft eine Sterilanzeige für einen Sterilbehälter gemäß dem Oberbegriff des Anspruchs 1.

### Hintergrund der Erfindung

Es ist bekannt, dass in der Medizin und insbesondere in der Chirurgie Sterilbehälter eingesetzt werden, die üblicherweise mit zur Sterilisation bestimmten chirurgischen Instrumenten bestückt/befüllt werden können. Während des Sterilisationsvorgangs verbleiben die Instrumente im Sterilbehälter und werden darin so stark und so lange mit einer bestimmten Temperatur erhitzt, bis an den chirurgischen Instrumenten gegebenenfalls befindliche Mikroorganismen sowie deren Sporen abgetötet sind. Um für einen Benutzer, wie beispielsweise für das Personal, das im Operationssaal die sterilen Instrumente verwenden möchte, sicherzustellen und anzuzeigen, dass die Instrumente tatsächlich einen Sterilisationsvorgang durchlaufen haben und deren Einsatz für einen Patienten hinsichtlich einer Infektionsgefahr unproblematisch sind, sind im Stand der Technik bereits unterschiedliche Ausführungsformen von Sterilanzeigen für Sterilbehälter vorgeschlagen worden.

### Stand der Technik

Beispielsweise ist aus der DE 33 16 141 A1 eine ausschließlich manuell zu betätigende Sterilanzeige in Form eines Verschlusssiegels für einen Sterilbehälter bekannt. Das Verschlusssiegel, das nur einem bestimmten Personenkreis zugänglich sein darf, zeigt in seinem an den Sterilbehälter angebrachten, unversehrten Zustand an, dass sich in dem Sterilbehälter sterilisierte Gegenstände, insbesondere chirurgische Instrumente befinden. Nachteilig an dieser Art von Sterilanzeige ist jedoch, dass das Verschlusssiegel nach dem Sterilisationsvorgang manuell angebracht, auf Unversehrtheit überprüft und vor dem Öffnen des Sterilbehälters wieder entfernt,
zumindest aber zerstört werden muss. Der Sterilisationsvorgang selbst muss für eine zuverlässige Beurteilung der Sterilität aufgrund des manuellen Anbringens des Verschlusssiegels auch genau überwacht werden. In anderen Worten ausgedrückt, gibt das Verschlusssiegel Auskunft darüber, dass der Inhalt des Sterilbehälters einem Sterilisationsprozess unterzogen wurde. Ob aber der Sterilisationsprozess korrekt und damit erfolgreich ausgeführt wurde, kann aus dem Verschlusssiegel selbst nicht abgeleitet werden.

Eine andere Art von Sterilanzeige ist beispielsweise aus der EP 0 412 571 B1 bekannt. Darin ist eine (intelligente) Sterilanzeige für einen Sterilbehälter beschrieben, die ein Anzeigelement zur Anzeige einer erfolgten Sterilisation des Inhalts des Sterilbehälters umfasst. Hierfür weist das Anzeigelement eine aus einer Gedächtnislegierung gefertigte Feder auf, die erst bei Erreichen oder beim Überschreiten einer vorbestimmten Temperatur, vorzugsweise der Sterilisationstemperatur (>90°C), über eine Federspannung verfügt. Durch die plötzlich anliegende Federspannung wird ein Verriegelungsorgan automatisch (ohne manuelles Zutun) in eine Schließstellung überführt, in der ersichtlich ist, dass eine Sterilisation stattgefunden hat (Quantitätshinweisfunktion) und auch korrekt durchgeführt wurde (Qualitätshinweisfunktion).

Weiter zeigt die DE 20 2009 010 210 U1 eine Überwachungseinrichtung für einen Sterilisationsbehälter mit einem Kontrollelement, welches zwischen einer Ruheposition und einer Aktivposition verlagerbar an einem Sterilisationsbehälter gelagert ist und welches nach einem Sterilisationsvorgang von der Ruheposition in die Aktivposition verlagert ist.

EP 0 849 429 A1 offenbart einen Safe mit einem Bolzenmechanismus. Als weitere Sicherheitsmaßnahme sind an der Tür thermische Verriegelungsvorrichtungen vorgesehen.

Nachteilig daran ist jedoch, dass für die Sterilanzeige eine vergleichsweise kostenintensive Spiralfeder aus einer Gedächtnislegierung (Formgedächtniselement) eingesetzt wird, sodass die Feder mehrmals verwendet werden muss. Es hat sich
jedoch gezeigt, dass die bekannte Feder nicht beliebig oft in ihre spannungsbehaftete Gedächtnisstellung gebracht werden kann, sondern hinsichtlich ihrer Lebenszeit und damit auch hinsichtlich der möglichen Sterilisationsvorgänge des Sterilbehälters erheblichen Beschränkungen unterworfen ist. Auch erweist sich die bekannte Sterilanzeige dahingehend als problematisch, als dass diese auf dem Prinzip eines vergleichsweise ungenauen/träge reagierenden, eine kreisförmige Stellbewegung ausführenden Drehfedersystems basiert. Dies kann zu falschen Anzeigeergebnissen führen. Schließlich muss die Sterilanzeige der EP 0 412 571 B1 beim Öffnen des Sterilbehälters manuell in ihre Öffnungsstellung zurück gestellt werden. Wird dies vergessen, lässt sich ein nachfolgender Sterilisationsprozess qualitativ nicht mehr dokumentieren.

### Kurzbeschreibung der Erfindung

Dem gegenüber liegt der Erfindung die Aufgabe zu Grunde, die Zuverlässigkeit einer Sterilanzeige für einen Sterilbehälter, unter Einsatz konstruktiv möglichst einfacher Mittel, zu verbessern. Ein Ziel ist es hierbei, dass die Sterilanzeige eine hohe Betriebssicherheit und einen möglichst einfachen konstruktiven Aufbau aufweist. Ein weiteres/anderes Ziel ist es, eine vergleichsweise hohe Anzahl von Sterilisierungsbeziehungsweise Aufbereitungszyklen zu gewährleisten.

Diese Aufgabe wird durch eine Sterilanzeige für einen medizinischen Sterilbehälter mit den Merkmalen des Anspruchs 1 gelöst. Vorteilhafte Weiterbildungen der Erfindung sind u. a. in den Unteransprüchen angegeben.

Der vorliegenden Erfindung liegt das Erfindungsprinzip/-konzept zugrunde, ein Formgedächtniselement in Gestalt einer axial wirkenden Druckfeder aus einem "Memory-Material" auszubilden, die bei Erreichen/Überschreiten einer vorbestimmten Temperatur eine Axialdruckkraft auf eine Anzeigevorrichtung/Anzeigeeinheit ausübt, die wiederum mittels einer (temperaturunabhängig arbeitenden) Rückstellfeder in Gegenrichtung des Formgedächtniselements vorgespannt/vorspannbar ist. Die beiden Federn sind so aufeinander abgestimmt, dass die bei der vorbestimmten Temperatur sich einstellende (maximale) Axialdruckkraft des Formgedächtniselements höher ist als die Federkraft der Rückstellfeder. Schließlich ist ein Rastmechanismus vorgesehen, der durch ein Öffnen/Schließen und/oder durch den Schließ-/Verriegelungsmechanismus eines Sterilcontainers (automatisch) mitbetätigt wird und so ausgebildet ist, dass er nach Schließen und/oder Verriegeln des Sterilcontainers die Anzeigeeinheit in einer Sterilposition hält, nachdem diese vom Formgedächtniselement in die Sterilposition bewegt worden ist und mit Öffnen und/oder Entriegeln des Sterilcontainers die Anzeigeeinheit freigibt, sodass die Rückstellfeder die Anzeigeeinheit in eine Unsterilposition rückbewegen kann.

Konstruktiv lässt sich das vorstehend beschriebene Erfindungskonzept grundsätzlich umsetzen, indem der Sterilcontainer mit einer Sterilanzeige ausgestattet wird/ist, bei der das Formgedächtniselement in Gestalt einer Schraubendruckfeder längs eines Schubelements (Schubstange) positioniert und mit diesem in Druckeingriff gebracht wird, auf das ferner eine (herkömmliche) Rückstellfeder (Rückstell-Schraubendruckfeder) eine dem Formgedächtniselement (immer, d.h. temperaturunabhängig) entgegenwirkende Rückstellkraft aufbringt, die kleiner bemessen ist, als eine (maximale) Druckkraft des Formgedächtniselements bei Erreichen/Überschreiten einer vorbestimmten Sterilisationstemperatur innerhalb des Containers. Das Schubelement ist wiederum mit der von Außen wahrnehmbaren Anzeigeeinheit gekoppelt oder bildet diese aus, die demzufolge in Abhängigkeit der Containerinnentemperatur und damit der vom Formgedächtniselement aktuell/temporär ausgeübten (maximalen) Druckkraft eine von Außerhalb des Containers wahrnehmbare (visuell, taktil, etc.) Sterilposition oder Unsterilposition annimmt. Dabei sei darauf hingewiesen, dass die Rückstellfeder nicht notwendiger Weise direkt auf die Schubstange einwirken muss, sondern mit der Anzeigeeinheit wirkverbunden sein kann und somit über die Anzeigeeinheit eine Rückstellkraft auf diese und damit auch auf das Schubelement ausübt.

Schließlich hat der Sterilcontainer einen Deckel/Zugangstüre und/oder einen vorzugsweise manuell betätigbaren Schließ-/Verriegelungsmechanismus für ein Öffnen/Entriegeln und Schließen/Verriegeln des Containers sowie einen Rastmechanismus vorzugsweise in Gestalt einer Rastfeder, der so ausgebildet und/oder platziert ist, dass er mit dem Deckel/Zugangstüre und/oder dem Schließ-/Verriegelungsmechanismus in Wirkeingriff bringbar ist oder steht, derart, dass der Rastmechanismus/die Rastfeder bei/durch Schließen und/oder Verriegeln des Sterilcontainers (durch eine Schließ- und/oder Verriegelungsbetätigung des Deckels/Zugangstüre und/oder des Schließ-/Verriegelungsmechanismus) zunächst nur in eine Rastfunktion (nicht Raststellung) (feder-) vorgespannt (also scharf gestellt) wird. Der Rastmechanismus bzw. die Rastfeder ist wiederum mit der Anzeigeeinheit vorzugsweise über das Schubelement in Wirkverbindung bzw. in die Sterilanzeige integriert, derart, dass mit Annehmen der Sterilposition (durch die Wirkung des Formgedächtniselements) die zunächst nur in ihre Rastfunktion vorgespannte (scharf gestellte) Rastmechanismus selbsttätig (durch die Vorspannung) in eine Raststellung gelangt (schnappt/auslöst), in der die Sterilanzeige/Anzeigeeinheit in der Sterilposition (dann unabhängig vom Formgedächtniselement) arretiert wird (auch dann, wenn danach die Druckkraft des Formgedächtniselements infolge eines Abkühlens des Container-Innenraums abnimmt). Wird der Schließ-/Verriegelungsmechanismus geöffnet/entriegelt und/oder der Deckel/Zugangstüre geöffnet, wird dadurch der Rastmechanismus bzw. die Rastfeder freigegeben und (durch dessen innere Vorspannung oder eine äußere Vorspannfeder) aus der Raststellung ausgerückt und gibt so die Sterilanzeige/Anzeigeeinheit frei, welche dann durch die Rückstellfeder in deren Unsterilposition bewegt wird.

Das Prinzip einer wiederverwendbaren Sterilanzeige an Sterilcontainern wurde bisher gemäß der vorstehenden Ausführungen nur in Verbindung mit spiralförmigen Thermobimetallen konstruktiv umgesetzt. Dabei wird die Umschaltung der Anzeige von einer Unsterilposition nach einer Sterilposition mittels einer Drehbewegung der Anzeige realisiert. Daraus ergeben sich auch aufgrund ggf. ungünstiger Hebelverhältnisse Ungenauigkeiten insbesondere im Ansprechverhalten der Sterilanzeige, die im schlimmsten Fall dazu führen können, dass die Anzeige auf die Sterilposition umschaltet, obgleich die geforderte Sterilisationstemperatur noch nicht ganz erreicht wurde.

Erfindungsgemäß ist aber gemäß einem Aspekt der Erfindung eine Druckfeder mit Memory-Effekt (Memory-Metal-Druckfeder, vorzugsweise aus NiTi-Metall) vorgesehen, die eine Axialbewegung über eine Mehrzahl von Schraubenwindungen realisiert. Dadurch lässt sich eine geringe Trägheit bei Temperaturwechseln erreichen, wodurch die Gefahr einer falschen Statusanzeige (Steril/Unsteril) verringert wird. Die (Schrauben-)Druckfeder ist in der Regel sehr zuverlässig und hat eine hohe Lebensdauer aufgrund einer vergleichsweise geringen Materialbelastung (pro Windung). Der gesamte Aufbau der erfindungsgemäßen Sterilanzeige ist kostengünstig, weil nur wenige Bauteile verbaut werden müssen.

Eine vorteilhafte Weiterbildung der Erfindung sieht die Anordnung eines Schubelements in Gestalt einer axial verschiebbaren Schubstange oder Schubplatte vor mit dem zumindest das Formgedächtniselement und vorzugsweise die Rückstellfeder in axialem Druckeingriff steht und welches eine von Außerhalb des Containers wahrnehmbare Anzeigeinheit in die Steril- und/oder Unsterilposition betätigt oder welches die Anzeigeeinheit trägt/stützt oder ausbildet. Damit reduzieren sich die absolut notwendigen Bauteile auf wenigstens 3 Teile, nämlich das Schubelement zur Kraftübertragung sowie zur Betätigung oder Ausbildung der Anzeige (-einheit) sowie die beiden gegensätzlich wirkenden Federn, die auf die Schubstange einwirken können. Diese Konstruktion ist besonders einfach, unanfällig gegen Erschütterungen (da die Anzeigeeinheit zwischen den Federn eingespannt ist) und kostengünstig.

Gemäß einem ggf. unabhängig zu beanspruchenden Aspekt der Erfindung kann es vorgesehen sein, dass der Rastmechanismus eine Rastfeder hat, die über ein vom Deckel/Zugangstüre und/oder Schließ-/Verriegelungsmechanismus betätigbares Stellglied in einen Rastfunktionszustand vorspannbar ist, sodass mit Erreichen der Sterilposition durch die Wirkung des Formgedächtniselements, die in ihre Rastfunktion vorgespannte Rastfeder selbsttätig in eine Raststellung gelangt, in der die Anzeigeeinheit in der Sterilposition arretiert/abgestützt wird auch dann, wenn danach die Druckkraft des Formgedächtniselements infolge eines Abkühlens des Container-Innenraums abnimmt. Die Rastfeder ist sehr einfach in ihrer Konstruktion und damit günstig sowie robust. Ohne elektrische Steuerung oder Sensorik lässt sich so auf rein mechanische Weise der Deckel/Zugangstüre und/oder der Schließ-/Verriegelungsmechanismus mit der Sterilanzeige koppeln, um die Qualität des Sterilisationsvorgangs bleibend (bis zum Entriegeln/Öffnen) zu dokumentieren. Außerdem lässt sich so erreichen, dass auf einfache Weise diese Dokumentation mit dem ersten Öffnen/Entriegeln des Containers nach dem Sterilisationsvorgang automatisch mechanisch gelöscht wird (Anzeigeeinheit schaltet automatisch in die Unsterilposition).

Vorzugsweise kann die Rastfeder ein separates Bauteil in Gestalt einer an einem Gehäuse der Sterilanzeige oder am Container montierten Blattfeder sein, welche im Rastfunktionszustand mit dem Schubelement in axialen Stützeingriff bringbar ist. Die Blattfeder kann vorzugsweise in Draufsicht die Form eines U annehmen mit zwei in Achsrichtung des Schubelements sich erstrechenden Schenkeln, zwischen den das Schubelement axial geführt ist, wodurch ein seitliches Abrutschen der Blattfeder von der Schubstange vermieden wird. Alternativ hierzu kann die Rastfeder ein separates Bauteil in Gestalt einer Stützplatte/-stange oder eines U-Bügels sein, welches senkrecht zum Schubelement über das Stellglied entgegen einer Feder verschiebbar an einem Gehäuse der Sterilanzeige oder am Container gelagert ist und im Rastfunktionszustand mit dem Schubelement axial (stirnseitig) in Stützeingriff bringbar ist. Weiter alternativ kann die Rastfeder als integrales Element der Sterilanzeige einen biegeelastischen Längsabschnitt des Schubelements bilden, der im Rastfunktionszustand bei einer Axialverschiebung des Schubelements in Richtung Sterilposition mit einem Anschlag oder Sperrstift am Sterilanzeigegehäuse oder am Container in Stützeingriff kommt.

Aus den vielen genannten Alternativen ist es ersichtlich, dass auch noch weitere Konstruktionen für einen Rastmechanismus in Abhängigkeit des Aufbaus des Containers und/oder des Schließmechanismus denkbar sind, solange der Rastmechanismus durch den Containerdeckel/-Zugangstür und/oder den Schließ-/Verriegelungsvorgang des Containers quasi scharf gestellt und mit Umschalten der Anzeigeeinheit in die Sterilposition ausgelöst wird, um die Anzeigeeinheit in der Sterilposition (mechanisch) zu halten. Vorteilhaft ist es dabei, dass diese Raststellung durch den Öffnungs-/Entriegelungsvorgang des Containers/Containerdeckels (quasi automatisch) aufgehoben wird, sodass die Anzeigeeinheit zwangsläufig wieder in deren Unsterilposition umschaltet. Dies geschieht vorzugsweise dadurch, indem die Kraft, welche durch den Deckel und/oder Schließmechanismus auf das Rastelement ausgeübt wird, um diese in die den Rastfunktionszustand zu spannen, mit Entriegeln und/oder Öffnen des Containers aufgehoben wird. Eine Gegendruckfeder kann dann das freigegebene Rastelement zurück in dessen Außerrastposition zurückbewegen.

Vorzugsweise ist das Formgedächtniselement eine NiTi-Memory-Metall-Schraubendruckfeder, die erst bei Erreichen oder Überschreiten einer Temperatur von ca. 90°C eine Federdruckkraft größer als die Rückstellkraft der Rückstellfeder infolge des temperaturbedingten Memory-Effekts erzeugt.

Erfindungsgemäß ist demzufolge auch ein Sterilcontainer zur Sterilisation von medizinischen, vorzugsweise chirurgischen Instrumenten vorgesehen mit einem vorzugsweise kastenförmigen Instrumentenaufnahmekörper, der mittels eines Containerdeckels/Zugangstüre verschließbar ist, wofür am Aufnahmekörper und/oder am Containerdeckel wenigstens ein manuell betätigbarer Schließ-/Verriegelungsmechanismus angeordnet sei kann oder ist und der mit der erfindungsgemäßen Sterilanzeige wie vorstehend ausgeführt ausgerüstet oder ausrüstbar ist.

Hierfür kann der Sterilcontainer vorzugsweise eine Montageschablone oder dergleichen Anschlusseinrichtung haben, die am Aufnahmekörper und/oder am Containerdeckel angeordnet/ausgebildet ist und an der die Sterilanzeige, vorzugsweise das die Sterilanzeige aufnehmende Anzeigegehäuse, wahlweise montierbar ist. Dadurch können Sterilcontainer je nach Kundenwunsch mit oder ohne die erfindungsgemäße Sterilanzeige ausgeliefert werden, wobei in einfacher Weise auch ein Nachrüsten des Containers oder ein Austauschen mit einer neuen Sterilanzeige möglich ist. Dabei ist es grundsätzlich möglich, die Sterilanzeige gehäuselos unmittelbar im Container zu verbauen indem die genannten Bauteile der Sterilanzeige unmittelbar am Container montiert werden.

### Figurenbeschreibung

Die Erfindung wird nachstehend anhand eines bevorzugten Ausführungsbeispiels unter Bezugnahme auf die begleitenden Figuren näher erläutert.
Fig. 1 zeigt eine in einem Sterilcontainer / Sterilcontainerdeckel (optional) eingebaute Sterilanzeige gemäß einem bevorzugten Ausführungsbeispiel der Erfindung in nicht scharf gestelltem Zustand,
Fig. 2 zeigt die Seitenansicht eines Sterilcontainer - Schließmechanismus mit der Sterilanzeige gemäß der Erfindung,
Fig. 3 zeigt eine konstruktiv alternative Ausgestaltung der Sterilanzeige gemäß der Erfindung,
Fig. 4 zeigt die Seitenansicht eines mittels eines Deckels geschlossenen Sterilcontainers mit der Sterilanzeige gemäß der Erfindung,
Fig. 5 zeigt die aufgeschnittene Seitenansicht eines geöffneten Sterilcontainers mit der Sterilanzeige gemäß der Erfindung in nicht scharf gestelltem Zustand,
Fig. 6 zeigt die aufgeschnittene Seitenansicht eines geöffneten Sterilcontainers mit der Sterilanzeige gemäß der Erfindung in scharf gestelltem Zustand und
Fig. 7 zeigt die Drauf- und Seitenansicht einer Rastfeder gemäß der vorliegenden Erfindung.

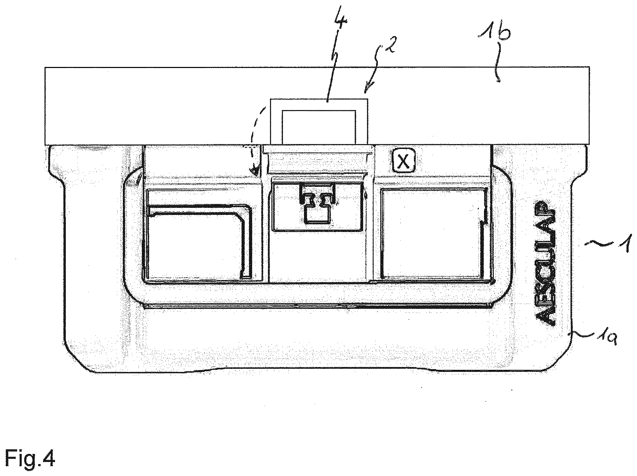

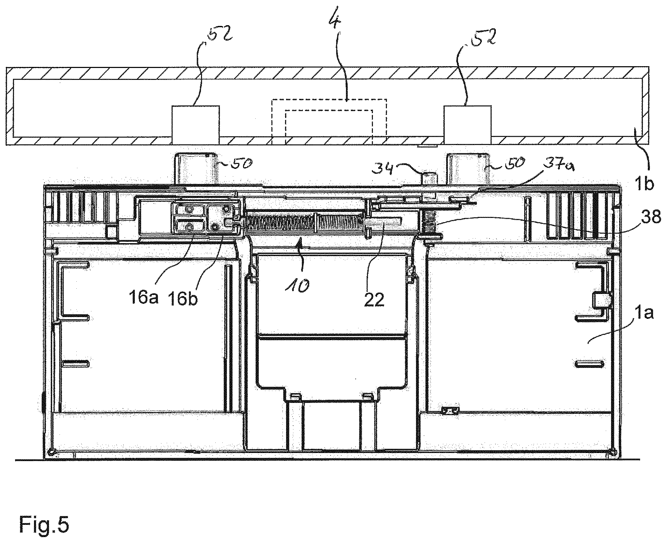

In den Fig. 4 und 5 ist ein Teil eines aus dem Stand der Technik beispielsweise gemäß der eingangs genannten Druckschriften bekannten Sterilcontainers schematisch dargestellt. Der Sterilcontainer 1 hat einen wannen- oder kastenförmigen InstrumentenAufnahmekörper 1a, der an zumindest einer Seite (Oberseite) mittels eines Containerdeckels 1b verschließbar ist. Am Deckel 1b und/oder am Aufnahmekörper 1a ist ein Schließ-/Verriegelungsmechanismus 2 bekannten Aufbaus angeordnet, mit einer manuell betätigbaren Handhabe (z.B. Schnalle, Hebel, etc.) 4, die gemäß der Fig. 4 beispielhaft durch einen schwenkbar an dem Deckel 1b gelagerten Bügel dargestellt ist, der mit einer Hinterschneidung an der Containerwanne 1a oder am Deckel 1b in Spanneingriff bringbar ist. Dabei sei ausdrücklich darauf hingewiesen, dass auch andere Konstruktionen für einen Schließ-/Verriegelungsmechanismus wie z.B. ein Schiebe- oder Drehriegel, ein Türschloss mit schwenkbaren Schnapphaken und dergleichen bekannte Mechanismen vorgesehen sein können. Auch ist es grundsätzlich denkbar, den Containerdeckel 1b gar nicht zu verriegeln sondern diesen ähnlich eines Frischhalte-Dosendeckels auf den freien Rand der Containerwanne 1a lösbar aufzupressen. Hierfür könnte am freien Rand der Containerwanne 1a z.B. eine Nut ausgeformt sein, in die der Deckel 1b randseitig eingedrückt werden kann.

Eine erfindungsgemäße Sterilanzeige 10 ist vorliegend als Container-integrale (fest eingebaute) Anordnung vorgesehen, wie dies insbesondere in der Fig. 5 dargestellt ist. Es ist aber auch denkbar, die Sterilanzeige 10 als externe/separate Baueinheit oder Anordnung vorzugsweise mit eigenem Gehäuse 12 (nicht näher gezeigt) vorzusehen, die an einer vordefinierten Montagestelle (Montagesockel) am Container 1 wahlweise an-/eingebaut werden kann.

Der Deckel 1b ist vorzugsweise an der Wanne 1a anscharniert oder frei abnehmbar und hat an wenigstens einer Auflagerkante zwei Ausnehmungen in Form von Sacklöchern oder Schlitzen 52, in die Passstifte oder Rippen 50 an der Wanne 1b eingreifen, um den Deckel 1b in geschlossener Lage formschlüssig zu positionieren. Alternativ kann die Wanne 1a natürlich auch mit stirnseitig umlaufenden Nuten ausgeformt sein, wie dies vorstehend bereits angedeutet wurde, in welche der Deckel 1b formschlüssig eingreifen kann.

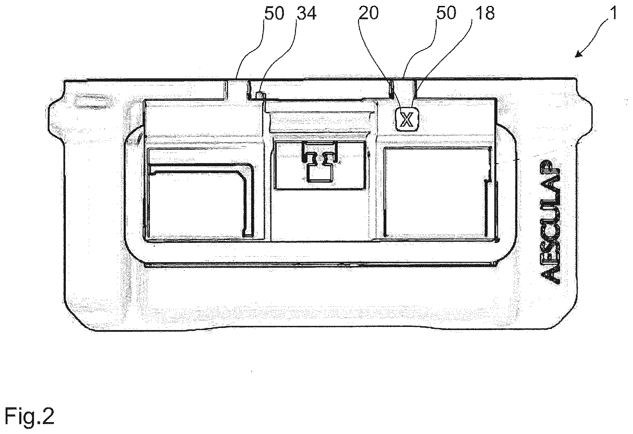

Die Sterilanzeige 10 hat eine Anzeigeeinheit 14, die von Außerhalb des Containers 1 vorzugsweise visuell wahrnehmbar wenigstens zwei Containerzustände anzeigen kann, nämlich einen Sterilzustand (entspricht einer Sterilposition der Anzeigeeinheit) und einen Unsterilzustand (entspricht einer Unsterilposition der Anzeigeeinheit). Hierfür hat die Anzeigeeinheit 14 einen Schieber 16 mit zwei in Schieberichtung getrennten Feldern 16a, 16b, die über ein Sichtfenster 18 im Container 1 (siehe Fig. 2) von außerhalb wahrnehmbar sind. In den Feldern 16a, 16b sind vorzugsweise Symbole 20 für Steril und Unsteril aufgebracht.

An der Anzeigeeinheit 16 ist ein Schubelement in Gestalt einer Betätigungsstange 22 angekoppelt, die zusammen mit der Anzeigeeinheit 16 axial verschiebbar am Container 1 oder am (nicht weiter dargestellten) Gehäuse 12 der Sterilanzeige 10 gelagert ist. Hierfür sind am Container 1/Anzeigegehäuse 12 wenigstens zwei Lagerbuchsen 24, 26 ausgeformt, in denen die Betätigungsstange 22 an deren beiden Endabschnitten gleitgelagert ist. Die Betätigungsstange 22 hat axial mittig einen Umfangskragen 28, der die Betätigungsstange 22 in zwei Axialabschnitte teilt. An einem der Anzeigeeinheit 16 zugewandten Axialabschnitt ist eine Rückstell-Schraubendruckfeder 30 um die Betätigungsstange 22 gewunden, die sich an der einen fixen Lagerbuchse 24 und an dem Umfangskragen 28 abstützt und so die Betätigungsstange 22 (konstant) weg von der anzeigeneinheitsseitigen Lagerbuchse 24 vorspannt. Dadurch wird die Anzeigeeinheit 16 (konstant) mit einer vordefinierten (temperaturunabhängigen) Rückstellkraft der Rückstell-Schraubendruckfeder 30 in deren Unsterilposition gehalten (Konstruktionslage).

An dem von der Anzeigeeinheit 16 abgewandten Axialabschnitt ist eine Memory-Metall-Schraubendruckfeder (Formgedächtniselement) 32 vorzugsweise aus NiTi-Memory-Metall angeordnet, die ebenfalls um die Betätigungsstange 22 gewunden ist und sich gegen den Umfangskragen 28 und die anzeigeneinheitsentfernte Lagerbuchse 26 abstützt und so der Rückstell-Schraubendruckfeder 30 (temperaturabhängig) entgegenwirkt.

Für die Memory-Metall-Schraubendruckfeder 32 wurde ein Material gewählt, das mit Überschreiten einer Temperatur von ca. 90°C seine ursprüngliche Form annimmt. D.h. dass die Memory-Metall-Schraubendruckfeder 32 so konstruiert ist, dass diese erst bei Überschreiten der vorbestimmten Sterilisationstemperatur von vorzugsweise 90°C eine Druckkraft auf die Betätigungsstange 22 ausübt, die höher ist als die (konstante/temperaturunabhängige) Rückstellkraft der Rückstell-Schraubendruckfeder 30. Infolge dessen wird die Betätigungsstange 22 mit Überschreiten der vorbestimmten Sterilisationstemperatur entgegen der Rückstell-Schraubendruckfeder 30 verschoben und dadurch die Anzeigeeinheit 16 in ihre Sterilposition geschaltet.

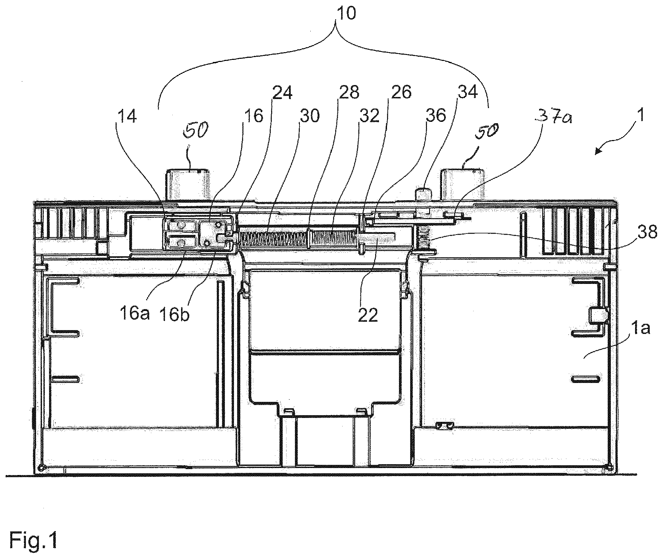

Wie aus der Fig. 1 und der Fig. 5 ferner zu entnehmen ist, hat die erfindungsgemäße Sterilanzeige 10 noch einen Rastmechanismus. Dieser hat vorliegend einen Betätigungsstift 34, der am Container 1 oder am Anzeigegehäuse 12 axial (in Schließrichtung des Deckels 1 b) verschiebbar gelagert ist, wobei dessen Axialrichtung somit im Wesentlichen senkrecht oder in einem Winkel zur Axialrichtung der Betätigungsstange 22 verläuft. Der Betätigungsstift 34 ist dabei entweder mit dem Schließmechanismus 2 wirkverbunden, derart, dass der Betätigungsstift 34 mit Betätigung des Schließmechanismus 2 axial verschoben wird; oder, wie im vorliegenden Beispiel konkret gezeigt, der Betätigungsstift 34 ragt axial aus dem Container 1 in Richtung hin zum Deckel 1b vor und wird bei Verschwenken/Aufsetzen des Deckels 1b in Schließposition axial in den Container 1 gedrückt. Dabei sei aber an dieser Stelle darauf hingewiesen, dass auch andere Kopplungsmöglichkeiten zwischen Deckel und Rastmechanismus (etwa im Bereich der Scharnierlagerung) oder zwischen Schließ-/Verriegelungsmechanismus und Rastmechanismus (etwa durch zwischengefügte Betätigungshebel) existieren.

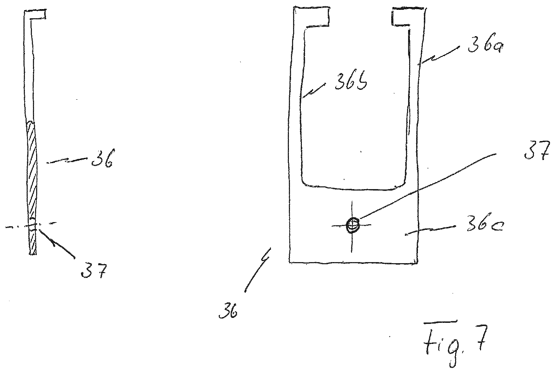

Am Betätigungsstift 34 ist eine Rastfeder 36 in Gestalt einer Federzunge fixiert. Diese Federzunge kann eine einfache Blattfeder sein oder in Draufsicht ein Profil aufweisen, wie es in der Fig. 7 angedeutet ist. Im letzteren Fall ist die Federzunge 36 in Draufsicht U-fömig ausgebildet mit zwei parallel beabstandeten Schenkeln 36a, 36b und einem Querbalken 36c, in dem ein Durchgangsloch 37 ausgeformt ist. Die freien Enden der Schenkel 36a, 36b sind zu Abstützflächen ca. 90° abgewinkelt. Das Durchgangsloch 37 hat einen solchen Durchmesser, dass der Betätigungsstift 34 hindurch gesteckt werden kann.

Schließlich ist eine Vorspannfeder 38, vorzugsweise Schraubenfeder um den Betätigungsstift 34 herum vorgesehen, die sich zum Einen am Container 1 oder dem Anzeigegehäuse 12 und zum Anderen an der Federzunge 36 abstützt und somit den Betätigungsstift 34 in Axialrichtung entgegen der Betätigungskraft durch den Schließ-/Verriegelungsmechanismus oder entgegen dem Containerdeckel (aus dem Container 1 heraus) vorspannt und dabei zunächst die Federzunge 36 gegen einen Containerseitigen / Gehäuseseitigen Anschlag 37a drückt. In dieser (Konstruktions-) Lage befindet sich die Federzunge 36 in einem Parallelabstand zur Betätigungsstange, wie dies insbesondere in der Fig. 5 dargestellt ist.

Die Federzunge 36 ist ferner so platziert, dass sie sich bei einer vordefinierten Axialverschiebung des Betätigungsstifts 34 entgegen der Vorspannfeder 38 (entsprechend dem aus dem Container 1 vorragenden Stiftabschnitt) sowie ausgelöst durch den Schließ-/Verriegelungsmechanismus oder das Aufsetzen des Deckels im Wesentlichen auf einer Achslinie zur Betätigungsstange 22 ausrichtet, d.h. sich hinter die eine Stirnseite der Betätigungsstange 22 legt und somit als axial wirkender Verschiebeanschlag für die Betätigungsstange 22 dient. Wird hingegen die Betätigungskraft auf den Betätigungsstift 34 weggenommen (z.B. durch Öffnen des Deckels 1b und/oder durch Betätigen des Verriegelungsmechanismus 2), drückt die Vorspannfeder 38 den Betätigungsstift 34 in seine Konstruktionslage gegen den Anschlag 37a zurück, in der sich die Federzunge (Rastfeder) 36 erneut im Wesentlichen parallel (im Parallelabstand) zur Betätigungsstange 22 positioniert.

Die Funktion der erfindungsgemäßen Anzeige wird nachfolgend anhand der Fig. 1 und 2 beschrieben.

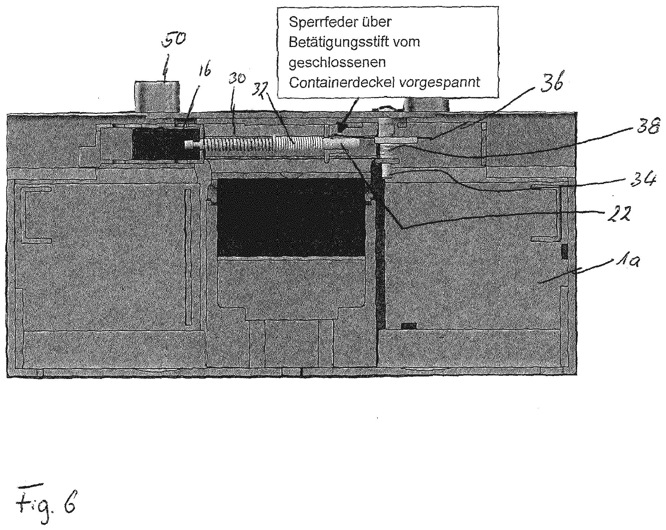

Durch Verriegeln des Container-Schließ-/Verriegelungsmechanismus (z.B. Verschwenken des Bügels 4) und/oder, wie dargestellt, durch Schließen des Deckels 1b wird zunächst der Betätigungsstift 34 entgegen der Vorspannfeder 38 axial (in den Container hinein) verschoben. Dabei kommt die Federzunge/Rastfeder 36 mit der Betätigungsstange 22 seitlich in Anlage und wird dadurch zunächst federelastisch gebogen/vorgespannt. Dieser Zustand ist in der Fig. 6 dargestellt. In diesem Zustand gemäß der Fig. 6 ist der Rastmechanismus vorerst nur in seine Rastfunktion (noch nicht Raststellung) scharf geschaltet. Im Fall einer U-förmigen Federzunge, wie sie in der Fig. 7 gezeigt ist, befindet sich die Betätigungsstange teilweise zwischen den federelastisch gebogenen Federzungen-Schenkeln 36a, 36b und wird so zwischen diesen Schenkeln 36a, 36b axial geführt.

Wird der geschlossene und/oder verriegelte Container 1 nun auf eine definierte Sterilisationstemperatur erhitzt, (größer/gleich 90°C) erzeugt die Memory-Metal-Schraubendruckfeder 32 eine vordefinierte Druckkraft auf die Betätigungsstange 22, die größer ist als die (konstante/temperaturunabhängige) Rückstellkraft der Rückstell-Schraubendruckfeder 30, wodurch die Betätigungsstange 22 in Richtung hin zur Anzeigeeinheit 16 axial verschoben wird. Dadurch wird die Anzeigeeinheit 16 mit verschoben und so aus der Unsterilposition in eine Sterilposition umgeschaltet, was gemäß der Fig. 2 durch das Sichtfenster 20 im Container 1 von Außen wahrnehmbar ist. Gleichzeitig schnappt die Federzunge 36 bzw. die Schenkel 36a, 36b infolge der Axialverschiebung der Betätigungsstange 22 federelastisch axial hinter die Stirnseite der Betätigungsstange 22. Im Fall der U-förmigen Federzunge 36 gemäß der Fig. 7 verhindern die Schenkel 36a, 36b dabei ein seitlichen Abrutschen von der Betätigungsstange 22 während deren Axialbewegung.

Kühlt nunmehr der Container 1 auf eine Temperatur unterhalb der Sterilisationstemperatur (beispielsweise 80°C) ab, reduziert sich die Federkraft der Memory-Metall-Schraubendruckfeder 32 auf einen Wert unterhalb der Rückstellfederkraft. Jedoch kann die Rückstell-Schraubendruckfeder 30 die Betätigungsstange 22 zunächst nicht (gemäß der Fig. 1 nach rechts) in deren Konstruktionslage verschieben, da diese von der Federzunge 36 der vom Deckel und/oder dem Verriegelungsmechanismus betätigten Rastvorrichtung als Axialanschlag abgestützt ist. Auf diese Weise verbleibt die Anzeigeeinheit 16 in der Sterilposition und dokumentiert so (auf mechanische Weise), dass der Sterilisationsvorgang korrekt durchgeführt wurde.

Wird nunmehr der Schließ-/Verriegelungsmechanismus 4 des Sterilcontainers 1 für ein Öffnen betätigt und/oder der Deckel 1 b geöffnet, kommt der daran wirkgekoppelte Betätigungsstift 34 des Rastmechanismus frei, wodurch dieser mittels der Vorspannfeder 38 axial in eine Außereingriffsstellung der Federzunge 36 bezüglich der Betätigungsstange 22 in Richtung hin zum Anschlag 37a verschoben wird (Konstruktionslage). Da somit die Abstützung der Betätigungsstange 22 durch die Federzunge 36 aufgehoben ist, wird diese durch die Rückstellfeder 30 axial verschoben, wodurch die Anzeigeeinheit 16 in deren Unsterilposition (siehe Fig. 2) umgeschaltet wird. Die Dokumentation des Sterilzustands des Containers 1 wird damit automatisch (zwangsläufig) durch erstmaliges Entriegeln des Schließ-/Verriegelungsmechanismus und/oder Öffnen des Deckels 1 b nach dem letzten Sterilisationsvorgang gelöscht und bleibt in der Unsterilposition auch dann, wenn der Container 1 erneut geschlossen und/oder verriegelt wird.

An dieser Stelle sei nochmals darauf hingewiesen, dass der Rastmechanismus nicht unmittelbar mit dem Schließ-/Verriegelungsmechanismus 4 wirkgekoppelt sein muss, sondern (mittelbar) auch durch den Containerdeckel 1b selbst betätigt werden kann, wobei auch eine (notwendige) Kombination aus Deckelschließvorgang und Verriegelungsvorgang zur Betätigung des Rastmechanismus denkbar wäre D.h. mit dem Öffnen und Schließen des Containerdeckels 1 b (ggf. in Kombination mit dem Ver-/Entriegeln des Verriegelungsmechanismus 2) wird der Betätigungsstift 34 des Rastmechanismus axial verschoben und dadurch der Rastmechanismus, wie in Fig. 6 gezeigt, scharf gestellt bzw. wie in der Fig. 5 gezeigt, ausgelöst (entschärft). Auch könnte die Federzunge 36 durch ein starres Stützelement ersetzt werden, welches dann schwenkbar am Betätigungsstift 34 gelagert und beispielsweise von der daran sich abstützenden Vorspannfeder 38 stabilisiert wird. Schließlich besteht auch die Möglichkeit, die Federzunge 36 direkt am Container 1 oder am Anzeigegehäuse 12 zu fixieren und den Betätigungsstift 34 als Drückelement vorzusehen, das die Federzunge 36 biegeelastisch in Richtung hin zu einer Stützposition mit der Betätigungsstange 22 vorspannt.

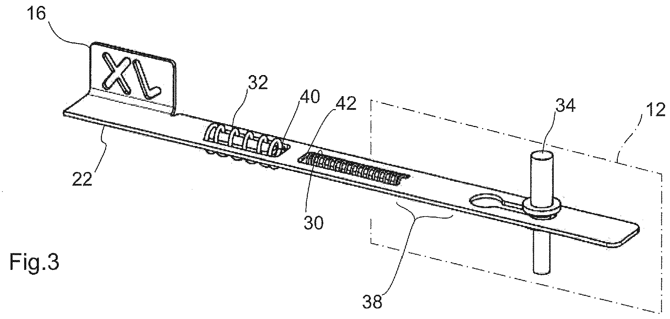

Schließlich ist in der Fig. 3 eine weitere alternative Ausgestaltung zu der vorstehend beschriebenen Konstruktion dargestellt.

In diesem Fall ist das Schubelement 22 nicht als Betätigungsstange sondern als flache Schubleiste (ggf. Stanzteil) ausgeformt mit zwei axial beabstandeten schlitzförmigen Aufnahmefenstern 40, 42 in die die beiden Federn (Memory-Metall-Schraubendruckfeder 32 und Rückstell-Schraubendruckfeder 30) eingesetzt sind, derart, dass sie sich an jeweils einer Endseite gegen die Schubleiste 22 und an jeweils einer anderen Endseite gegen den Container 1/Anzeigegehäuse 12 (in Fig. 3 nur schematisch gezeigt) abstützen, wie dies auch anhand der Fig. 1 beschrieben wurde. Ferner ist in Fig. 3 die Schubleiste 22 voezugsweise nicht mit der Anzeigeeinheit 16 gekoppelt sondern trägt diese bzw. bildet diese einstückig aus.

Schließlich ist die Vorspannfeder 38 des Rastmechanismus nicht ein separates Bauteil, wie dies in der Fig. 1 gezeigt ist, sondern die Schubleiste 22 hat einen biegeelastischen Längsabschnitt 38, der eine Art Federzunge zur Ausbildung der Vorspannfeder darstellt.

Wird demnach der Betätigungsstift 34 durch den Containerdeckel und/oder den Schließ-/Verriegelungsmechanismus axial aus seiner Konstruktionslage verschoben, wird dadurch die Schubleiste 22 an ihrem federelastischen Abschnitt 38 in Biegerichtung vorgespannt. Verschiebt sich nunmehr die Schubleiste 22 durch die Federkraft der Memory-Metall- Schraubendruckfeder 32 bei Erreichen/Überschreiten der Sterilisationstemperatur, schnappt der federelastische Abschnitt 38 der Schubleiste 22 hinter einen (nicht gezeigten) Anschlag am Container 1 oder Anzeigegehäuse 12, und stützt so die Schubleiste 22 gegen die Rückstellfeder 30 axial ab. Dadurch verbleibt diese in der Sterilposition auch bei Abfall der Temperatur unter die Sterilisationstemperatur (Abfall der Federkraft der Memory-Metall-Schraubendruckfeder 32).

Wird schließlich durch Entriegeln und/oder Öffnen des Containers 1 der Betätigungsstift 34 axial freigegeben, löst der biegeelastische Abschnitt 38 der Schubleiste 22 aus, sodass die Rückstellfeder 32 die Schubleiste 22 axial aus der Steril- in die Unsterilposition zurück verschieben kann. Damit ist auch hier die Anzeige des Sterilzustands des Containers 1 bzw. dessen Inhalts gelöscht.

Offenbart wird abschließend eine Sterilanzeige 10 für einen Sterilcontainer 1 mit einem Formgedächtniselement 32 in Gestalt einer axial wirkenden Druckfeder vorzugsweise der Schraubenfederbauart, die erst bei Erreichen oder Überschreiten einer vorbestimmten Sterilisationstemperatur eine definierte Axialdruckkraft auf eine Anzeigeeinheit 16 ausübt, die wiederum mittels einer temperaturunabhängig arbeitenden Rückstellfeder 12 vorzugsweise der Schraubenfederbauart in Gegenrichtung des Formgedächtniselements 32 mit einer Rückstellkraft kleiner als die definierte Axialdruckkraft vorgespannt ist und mit einem Rastmechanismus, der durch einen manuell betätigbaren Schließ- oder Verriegelungsmechanismus des Sterilcontainers und/oder durch den Containerdeckel und/oder einem darn angebrachten Element (z.B. Hebel, Taste, etc.) betätigbar ist und so ausgebildet ist, dass er nach oder mit Schließen und/oder Verriegeln des Sterilcontainers 1 die Anzeigeeinheit 16 in einer Sterilposition hält, nachdem diese vom Formgedächtniselement 32 in die Sterilposition bewegt worden ist und mit Öffnen und/oder Entriegeln des Sterilcontainers 1 unterhalb der Sterilisationstemperatur die Anzeigeeinheit 16 freigibt, worauf die Rückstellfeder 30 die Anzeigeeinheit 16 in eine Unsterilposition zurück bewegt.

## Patentansprüche

1. Sterilanzeige eines Sterilcontainers (1) mit einem Formgedächtniselement (32) in Gestalt einer axial wirkenden Druckfeder aus einem Memory-Material, wobei die axial wirkende Druckfeder, der Schraubenfederbauart, erst bei Erreichen oder Überschreiten einer vorbestimmten Sterilisationstemperatur eine definierte Axialdruckkraft auf eine Anzeigeeinheit (16) ausübt, die wiederum mittels einer temperaturunabhängig funktionierenden Rückstellfeder (30), in Gegenrichtung des Formgedächtniselements (32) mit einer Rückstellkraft kleiner als die definierte Axialdruckkraft vorgespannt ist und mit einem Rastmechanismus (34,36, 38), der durch einen manuell betätigbaren Schließ- oder Verriegelungsmechanismus (2), durch einen Containerdeckel (1b) des Sterilcontainers (1) oder einem daran angebrachten Element betätigt wird und so ausgebildet ist, dass er durch Schließen und/oder Verriegeln des Sterilcontainers (1) die Anzeigeeinheit (16) in einer Sterilposition hält, nachdem diese vom Formgedächtniselement (32) in die Sterilposition bewegt worden ist und durch Öffnen oder Entriegeln des Sterilcontainers (1) unterhalb der Sterilisationstemperatur die Anzeigeeinheit (16) freigibt, worauf die Rückstellfeder (30) die Anzeigeeinheit (16) in eine Unsterilposition rückbewegt.

2. Sterilanzeige nach Anspruch 1, **gekennzeichnet durch** ein Schubelement in Gestalt einer axial verschiebbaren Schubstange oder Schubleiste (22) mit dem zumindest das Formgedächtniselement (32) und vorzugsweise die Rückstellfeder (30) in axialem Druckeingriff steht und welches die von Außerhalb des Containers (1) wahrnehmbare Anzeigeinheit (16) in die Steril- oder Unsterilposition betätigt oder welches die Anzeigeeinheit (16) trägt oder ausbildet.

3. Sterilanzeige nach Anspruch 1 oder 2, **dadurch gekennzeichnet, dass** sich das Formgedächtniselement (32) und die Rückstellfeder (30) an einem Gehäuse (12) der Sterilanzeige (10) oder am Sterilcontainer (1) als Widerlager abstützen.

4. Sterilanzeige nach einem der vorstehenden Ansprüche, **dadurch gekennzeichnet, dass** der Rastmechanismus ein Stützelement, vorzugsweise in Form einer Rastfeder (36), hat, das über ein vom Schließ-/Verriegelungsmechanismus oder vom Containerdeckel (1b) betätigbares Stellglied (34) in einen Rastfunktionszustand vorspannbar ist, sodass mit Erreichen der Sterilposition das in seine Rastfunktion vorgespannte Stützelement (36) selbsttätig in eine Raststellung gelangt, in der die Anzeigeeinheit (16) in der Sterilposition arretiert wird auch dann, wenn danach die Druckkraft des Formgedächtniselements (32) infolge eines Abkühlens des Container-Innenraums abnimmt.

5. Sterilanzeige nach Anspruch 4, **dadurch gekennzeichnet, dass** das Stützelement (36) ein separates Bauteil in Gestalt einer an einem Gehäuse (12) der Sterilanzeige (10) oder am Container (1) montierten Blattfeder, vorzugsweise in U-Form, ist, welche im Rastfunktionszustand, in der sie sich seitlich am Schubelement (22) federelastisch anlegt mit dem Schubelement (22) stirnseitig in Stützeingriff bringbar ist, nachdem das Schubelement (22) vom Formgedächtniselement (32) axial verschoben wurde.

6. Sterilanzeige nach Anspruch 4, **dadurch gekennzeichnet, dass** das Stützelement (36) ein separates Bauteil in Gestalt einer Stützplatte/-stange ist, welche senkrecht zum Schubelement (22) über das Stellglied (34) entgegen einer Feder (38) verschiebbar an einem Gehäuse (12) der Sterilanzeige (10) oder am Container (10) gelagert ist und im Rastfunktionszustand mit dem Schubelement (22) axial in Stützeingriff bringbar ist, nachdem das Schubelement (22) vom Formgedächtniselement (32) axial verschoben wurde.

7. Sterilanzeige nach Anspruch 4 **dadurch gekennzeichnet, dass** das Stützelement in Form der Rastfeder (36) als integrales Element der Sterilanzeige (10) einen biegeelastischen Längsabschnitt des Schubelements (22) bildet, der im Rastfunktionszustand bei einer Axialverschiebung des Schubelements (22) in Richtung Sterilposition mit einem Anschlag oder Sperrstift am Sterilanzeigegehäuse (12) oder am Container (1) in Stützeingriff kommt.

8. Sterilanzeige nach einem der vorstehenden Ansprüche, **dadurch gekennzeichnet, dass** das Formgedächtniselement (32) eine NiTi-Memory-Metall-Schraubendruckfeder ist, die erst bei Überschreiten einer Temperatur von ca. 90°C eine Federdruckkraft größer als die Rückstellkraft der Rückstellfeder infolge des temperaturbedingten Memory-Effekts erzeugt.

9. Sterilcontainer zur Sterilisation von medizinischen Instrumenten mit einem kastenförmigen Instrumentenaufnahmekörper (1a), der mittels eines Containerdeckels (1b) verschließbar ist, wofür am Aufnahmekörper (1a) oder am Containerdeckel (1b) wenigstens ein manuell betätigbarer Schließ-/Verriegelungsmechanismus angeordnet ist, **gekennzeichnet durch** eine Sterilanzeige (10) gemäß einem der Ansprüche 1 bis 8.

10. Sterilcontainer nach Anspruch 9, **gekennzeichnet durch** eine Montageschablone (50), die am Aufnahmekörper (1a) oder am Containerdeckel (1b) angeordnet ist und an der die Sterilanzeige (10) wahlweise montierbar ist.

## Claims

1. A sterilization indicator of a sterile container (1) comprising a shape-memory element (32) in the form of an axially acting compression spring made of memory material, wherein
the axially acting compression spring of the helical spring type, when a predetermined sterilization temperature is reached or exceeded, subjects an indicating unit (16) to a defined axial compression force, said indicating unit in turn being biased by means of a restoring spring (30) functioning independently of temperature in a counter-direction of the shape-memory element (32) by way of a restoring force which is smaller than the defined axial compressive force, and comprising a latching mechanism (34, 36, 38) which is actuated by a manually operable closing or locking mechanism (2), by a container cover (1b) of the sterile container (1) or an element attached thereto and is designed such that by closing and/or locking of the sterile container (1) it retains the indicating unit (16) in a sterile position, once said unit has been moved into the sterile position by the shape-memory element (32), and by opening or unlocking of the sterile container (1) below the sterilization temperature it releases the indicating unit (16), whereupon the restoring spring (30) moves the indicating unit (16) back into an unsterile position.

2. The sterilization indicator according to claim 1, **characterized by** a push element in the form of an axially movable push rod or push strip (22) with which at least the shape-memory element (32) and preferably the restoring spring (30) are in axial compression engagement and which actuates the indicating unit (16) perceptible from outside of the container (1) into the sterile position or unsterile position or which supports or forms the indicating unit (16).

3. The sterilization indicator according to claim 1 or 2, **characterized in that** the shape-memory element (32) and the restoring spring (30) rest on a housing (12) of the sterilization indicator (10) or on the sterile container (1) forming a counter-bearing.

4. The sterilization indicator according to any one of the preceding claims, **characterized in that** the latching mechanism includes a supporting element preferably in the form of a latch spring (36) adapted to be biased via an actuator (34) operable by the closing/locking mechanism or by the container cover (1b) into a latching function state so that upon reaching the sterile position the support element (36) biased into its latching function automatically adopts a latching position in which the indicating unit (16) is locked in the sterile position even when afterwards the compressive force of the shaped-memory element (32) decreases as the interior of the container cools down.

5. The sterilization indicator according to claim 4, **characterized in that** the support element (36) is a separate component in the form of a leaf spring, preferably in U-shape, mounted on a housing (12) of the sterilization indicator (10) or on the container (1), said leaf spring in the latching function state in which it is resiliently adjacent to the side of the push element (22) being adapted to be brought in supporting engagement with the push element (22) at the end face, once the push element (22) has been axially displaced by the shape-memory element (32).

6. The sterilization indicator according to claim 4, **characterized in that** the support element (36) is a separate component in the form of a support plate/rod which is movably supported perpendicularly to the push element (22) via the actuator (34) against a spring (38) on a housing (12) of the sterilization indicator (10) or on the container (1) and in the latching function state is adapted to be brought in axial support engagement with the push element (22), once the push element (22) has been axially displaced by the shape-memory element (32).

7. The sterilization indicator according to claim 4, **characterized in that** the support element in the form of the latch spring (36) as an integral element of the sterilization indicator (10) forms a resilient longitudinal portion of the push element (22) which in the latching function state upon axial displacement of the push element (22) in the direction of the sterile position enters into support engagement with a stop or locking pin at the sterilization indicator housing (12) or at the container (1).

8. The sterilization indicator according to any one of the preceding claims, **characterized in that** the shape-memory element (32) is a NiTi memory-metal helical compression spring which, when a temperature of approx. 90°C is exceeded, produces a spring-loaded force higher than the restoring force of the restoring spring due to the temperature-dependent memory effect.

9. A sterile container for sterilization of medical instruments comprising a box-shaped instrument holding body (1a) closable by means of a container cover (1b) for which purpose at least one manually operable closing/locking mechanism is arranged on the holding body (1a) or on the container cover (1b), **characterized by** a sterilization indicator (10) according to any one of the claims 1 to 8.

10. The sterile container according to claim 9, **characterized by** a mounting pattern (50) which is arranged at the holding body (1a) or at the container cover (1b) and on which the sterilization indicator (10) may optionally be mounted.

## Revendications

1. Indicateur de stérilité d'un récipient stérile (1) avec un élément à mémoire de forme (32) sous la forme d'un ressort de compression agissant axialement, en un matériau mémoire, dans lequel le ressort de compression agissant axialement de type ressort hélicoïdal exerce uniquement lorsqu'une température de stérilisation prédéterminée est atteinte ou dépassée, une compression axiale définie sur une unité d'affichage (16) qui est lui-même précontraint au moyen d'un ressort de rappel (30) fonctionnant indépendamment de la température, dans la direction opposée de l'élément à mémoire de forme (32) avec une force de rappel inférieure à la force de pression axiale définie et avec un mécanisme à cliquet (34, 36, 38) qui est actionné par un mécanisme de fermeture et de verrouillage (2) pouvant être actionné manuellement par le biais d'un couvercle de récipient (1b) du récipient stérile (1) ou d'un élément monté sur celui-ci et est conçu de telle sorte que par la fermeture et/ou le verrouillage du récipient stérile (1), il maintienne l'unité d'affichage (16) dans une position stérile, après que celui-ci a été déplacé de l'élément à mémoire de forme (32) dans la position stérile et libère l'unité d'affichage (16) par l'ouverture ou le déverrouillage du récipient stérile (1) en dessous de la température de stérilisation, après quoi le ressort de rappel (30) ramène l'unité d'affichage (16) dans une position non stérile.

2. Indicateur de stérilité selon la revendication 1, **caractérisé par** un élément de poussée sous la forme d'une barre de poussée ou d'une réglette de poussée (22) décalable axialement avec au moins l'élément à mémoire de forme (32) et de préférence le ressort de rappel (30) est en prise par pression axiale et qui actionne l'unité d'affichage (16) observable de l'extérieur du récipient (1) dans la position stérile ou non stérile et qui porte ou constitue l'unité d'affichage (16).

3. Indicateur de stérilité selon la revendication 1 ou 2, **caractérisé en ce que** l'élément à mémoire de forme (32) et le ressort de rappel (30) s'appuient sur un logement (12) de l'indicateur de stérilité (10) ou sur le récipient stérile (1) en tant que butée.

4. Indicateur de stérilité selon l'une des revendications précédentes, **caractérisé en ce que** le mécanisme à cliquet a un élément d'appui, de préférence sous la forme d'un ressort d'encliquetage (36) qui est précontraint par un mécanisme de fermeture/verrouillage ou un actionneur (34) pouvant être actionné par le couvercle de récipient (1b) en un état de fonction d'encliquetage, de sorte qu'en atteignant la position stérile, l'élément d'appui (36) précontraint dans sa fonction d'encliquetage parvienne automatiquement dans une position d'encliquetage dans laquelle l'unité d'affichage (16) est arrêtée dans la position stérile lorsqu'ensuite la force de compression de l'élément à mémoire de forme (32) diminue en conséquence d'un refroidissement de l'espace intérieur du récipient.

5. Indicateur de stérilité selon la revendication 4, **caractérisé en ce que** l'élément d'appui (36) est un composant séparé sous la forme d'un ressort à lame monté sur un logement (12) de l'indicateur de stérilité (10) ou sur le récipient (1), de préférence en forme de U, qui, à l'état de fonction d'encliquetage dans lequel il est placé élastiquement, latéralement par rapport à l'élément de poussée (22), peut être amené à l'avant en contact par appui avec l'élément de poussée (22), après que l'élément de poussée (22) a été décalé axialement de l'élément à mémoire de forme (32).

6. Indicateur de stérilité selon la revendication 4, **caractérisé en ce que** l'élément d'appui (36) est un composant séparé sous la forme d'une plaque/barre d'appui qui est placée de façon à pouvoir être décalée perpendiculairement à l'élément de poussée (22) par l'actionneur (34) à l'encontre d'un ressort (38) sur un logement (12) de l'indicateur de stérilité (10) ou sur le récipient (10) et peut être amené en contact par appui axialement à l'état de fonction d'encliquetage avec l'élément de poussée (22) après que l'élément de poussée (22) de l'élément à mémoire de forme (32) a été décalé axialement.

7. Indicateur de stérilité selon la revendication 4, **caractérisé en ce que** l'élément d'appui sous la forme du ressort d'encliquetage (36) en tant qu'élément d'un seul tenant de l'indicateur de stérilité (10) forme un segment longitudinal élastique en flexion de l'élément de poussée (22) qui, à l'état de fonction d'encliquetage vient en contact par appui lors d'un décalage axial de l'élément de poussée (22) en direction de la position stérile avec une butée ou une tige de blocage sur le logement de l'indicateur de stérilité (12) ou sur le récipient (1).

8. Indicateur de stérilité selon l'une des revendications précédentes, **caractérisé en ce que** l'élément à mémoire de forme (32) est un ressort de compression hélicoïdal à mémoire NiTi qui génère, uniquement lors d'un dépassement de température d'environ 90 °C, une force de compression de ressort supérieure à la force de rappel du ressort de rappel en conséquence de l'effet de mémoire en fonction de la température.

9. Récipient stérile, pour la stérilisation d'instruments médicaux avec un corps de réception d'instruments (1a) en forme de boîte qui peut être fermé au moyen d'un couvercle du récipient (1b), ce pourquoi est disposé sur le corps de réception (1a) ou sur le couvercle du récipient (1b) au moins un mécanisme de fermeture/verrouillage pouvant être actionné manuellement, **caractérisé par** un indicateur de stérilité (10) selon l'une des revendications 1 à 8.

10. Récipient stérile selon la revendication 9, **caractérisé par** un gabarit de montage (50) qui est disposé sur le corps de réception (1a) ou sur le couvercle du récipient (1b) et qui peut être monté sélectivement sur l'indicateur de stérilité (10).
